Europäisches Patentamt

(19) European Patent Office

Office européen des brevets

(11) Publication number: **0 198 708 B1**

# EUROPEAN PATENT SPECIFICATION

(45) Date of publication of patent specification: **12.06.91**  (51) Int. Cl.⁵: **A61M 15/00, A61M 16/20**

(21) Application number: **86302792.6**

(22) Date of filing: **15.04.86**

(54) Portable oxygen inhaler.

(30) Priority: **15.04.85 JP 78264/85**
**11.10.85 JP 154352/85**
**19.11.85 JP 176841/85**

(43) Date of publication of application:
**22.10.86 Bulletin 86/43**

(45) Publication of the grant of the patent:
**12.06.91 Bulletin 91/24**

(84) Designated Contracting States:
**DE FR GB IT NL**

(56) References cited:
**GB-A- 971 161**
**GB-A- 1 039 528**
**GB-A- 1 069 917**
**US-A- 3 776 227**

(73) Proprietor: **Nippon Tansan Gas Co., Ltd.**
**32-26, Aoi 3-Chome**
**Adachi-ku Tokyo-to, 120(JP)**

(72) Inventor: **Fumino, Ichiro**
**7-13, 2-chome, Sagimiya**
**Nakano-Ku Tokyo(JP)**

(74) Representative: **Ablett, Graham Keith et al**
**F.J. Cleveland & Company 40-43 Chancery**
**Lane**
**London WC2A 1JQ(GB)**

## Description

The present invention relates to portable oxygen inhalers and more particularly to ones which are made in good unity co-axially arranging a valve pin and an adjusting mechanism and which let the adjusting mechanism adjust a secondary gas pressure and also quantity of emission of the gas, relating a pressure adjusting mechanism to the valve pin.

A heart deseased patient, his air intake ability being low, tends to be in lack of oxygen, which causes a spasmodic symptom. In such a case a quick treatment with an oxygen inhaler is necessary for him. Having taken immoderate exercise, even a healthy man needs a quantity of oxygen. If oxygen supply is not sufficient, it takes long time for his recovery from fatigue.

Inhalers known publically at present are generally bulky and difficult to use as a pressure reducing mechanism is provided at right angles with the axial line of a cartridge piercing device.

GB 1039528 discloses a fluid pressure regulating device of a type primarily intended to dispense oxygen from an oxygen cylinder. The device is in the form of a control head for controlling the pressure or flow of fluid from a container for fluid under pressure in association with a lower valve body attached to the outlet port of the container incorporating a valve operable by the control head.

An object of the present invention is to provide portable oxygen inhalers which are compact and used with ease, and which can adjust quantity of gas flow through an orifice, by adjusting a secondary pressure by an adjusting mechanism with a valve pin.

According to the present invention there is provided a portable oxygen inhaler comprising on the axial line of a cylindrical body a cartridge access device, a valve housing having a passage for the gas connecting a primary and a secondary spaces on each side of a valve pin, which is movably inserted in the passage and which has a shutting off portion which tends under the pressures of a spring and gas to shut off, said passage with a co-operative side of a seal member, an adjusting mechanism for the secondary pressure space being in contact at one end of the adjusting mechanism with an end of said valve pin in said secondary space, said secondary space being connected to a spout through an orifice, the quantity of flow of the gas through said orifice being adjustable by altering the secondary pressure with said adjusting mechanism characterised in that said cartridge access device has a needle for piercing the cartridge and a packing which surrounds the needle, said valve housing is mounted in said cartridge access device and a conduit connects the primary gas pressure space with the side of the seal member opposite to said co-operative side whereby the opposite side is subjected to the primary gas pressure so as to equalise the gas pressure on the seal member when the valve is open.

Accompanying drawings show the embodiments of the present invention by way of illustration only, in which:

Figure 1 is an elevation in split section showing one embodiment of the invention when not in use and partially in use,

Figure 2 is a plan view, of Figure 1,

Figure 3 is a side view of another embodiment of a packing, half in section,

Figure 4 is a plan view of a half of the packing shown in Figure 3,

Figure 5 is a partial elevation in section showing a cartridge of another embodiment using the packing,

Figure 6 shows a partial sectional elevation of a further embodiment of the invention which uses a push button,

Figure 7 shows a partial sectional elevation of a further embodiment of the invention which uses a lever,

Figure 8 is a section along the line G-G of Figure 7,

Figure 9 is a partial sectional elevation of a further embodiment of the invention which uses a push rod,

Figure 10 is a section along the line H-H of Figure 9,

Figure 11 shows a partial sectional elevation of a further embodiment of the invention which uses a cam lever,

Figure 12 shows a partial sectional elevation of a further embodiment of the invention which uses a push button,

Figure 13 is a partial sectional view of a further embodiment of the invention which has a leader for a primary gas pressure to a valve housing to a seal member,

Figure 14 is a plan view of the valve housing,

Figure 15 shows, half in section, an elevation of the valve housing in Figure 14,

Figure 16 is a plan view of another embodiment of a housing, and

Figure 17 shows, half in section, an elevation of the valve housing shown in Figure 16.

The embodiments of the present invention are hereinafter detailed with reference to the drawings. Throughout the descriptions and the drawings, the same symbols designate the same or the similar portions.

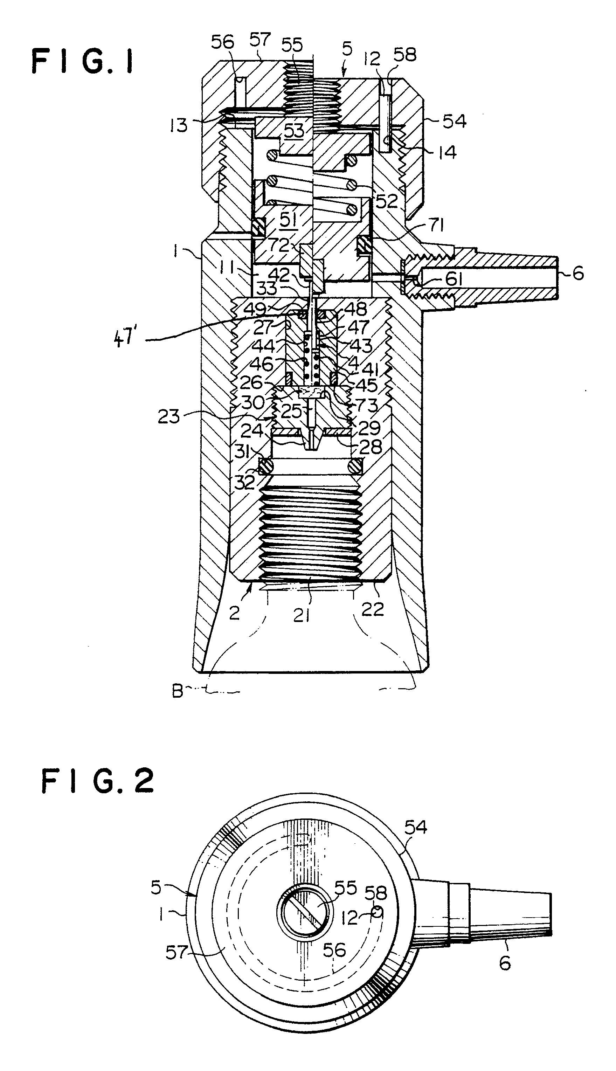

In Figures 1 and 2, 1 is a cylindrical body. On the axial line of the body 1, a cartridge piercing device 2, a valve pin 4 and an adjusting mechanism 5 are aligned. The cartridge piercing device 2 comprises a coupling screw 21 for a cartridge B and a needle block 23, which projects a needle 24 downward. The coupling screw 21 is formed at the inside of the lower end of a screw cylinder 22 screwed into the lower portion of the body 1. The needle block 23 is screwed into the screw cylinder 22 to be pressed against a step 26 which is at the lower end of a room 27, which is above the coupling screw and has a reduced diameter. A gas passage 25 is formed through the needle block 23 inclusive of the needle 24. A packing 28 is set on the surface of the needle block 23 around the needle 24.

An expanded portion 29 at the upper end of the gas passage 25 is filled with a filter 30. An O-ring 31, which is to be pressed around the neck portion of the cartridge B, is inserted in an annular groove 32 above the coupling screw 21. The room 27 is connected by a more reduced passage 33 with a secondary space 11 over the screw cylinder 22.

The valve pin 4 comprises a flange 41 at the lower end thereof, a passing portion 42 continuing from a shutting off portion 43 and a boundary portion. The flange 41 is loosely fitted in a passage 44, which is to be a primary pressure side of the gas and which is formed in a valve housing 45 fitted in the room 27 of the screw cylinder 22. The valve pin 4 is always urged upward by a spring 46 installed between the flange 41 and the filter 30, but can not move upward when the flange 41 comes into contact with a step 47 at the lower end of a reduced diameter passage 48, whose diameter is of the same length as that of the passage 33. The passage 48 is formed in the valve housing 45.

The passage 33 and 44 are connected by the passage 48. An O-ring 49 is fitted in an annular groove 47' in the upper end of the valve housing 45 to surround the reduced diameter passage 48. When the flange 41 comes into contact with the step 47, the shutting off portion 43 fits closely to the O-ring and stops gas flow. When the flange 41 parts from the step 47, the gas flows from the passage 48 into the passage 33. Thus the connection between the primary and the secondary pressure sides is formed or shut off by the valve pin. The boundary portion between the shutting off portion 43 and the passing portion 42 is favorably inclined so as not to damage the O-ring during the movement of the valve pin 4. The passing portion 42 may be provided with a flange 49' at the upper end thereof.

The adjusting mechanism 5 can adjust the pressure of the gas from the cartridge. The adjusting mechanism 5 comprises a piston 51 which contacts with the top of the valve pin 4 at its lower plane and which fits freely slidably in the secondary space 11 in the body 1, a pressure adjusting spring 52 which is installed between the piston and an upper spring seat 53, a dial 54 which engages with the upper portion of the body 1 with the threads and is movable upward and downward by rotating, and a pressure adjusting screw 55 which engages with the threads of dial 54 and contacts with the top of the spring seat 53 at its lower end. The pressure adjusting spring 52 of the type is used, which the compression force becomes maximum when the dial 54 is rotated into the lowermost position, by removing the load by the pressure adjusting screw 55, and which loses the compression force when the dial 54 is rotated back uppermost. To the secondary space 11, a spout 6 with an orifice 61 is provided.

When the pressure in the secondary space 11 varies, the pressure adjusting spring 52 is compressed and the piston 51 moves. The valve pin 4 also moves in the same direction as the piston 51, and it stops the gas by shutting off the reduced diameter passage 48.

When the valve pin 4 locates low, it must rise long stroke and compress heavily the pressure adjusting spring 52, to shut off the gas and reduce the pressure in the secondary space 11, then a large quantity of gas flows throught the orifice 61. On the contrary, when the valve pin 4 locates high, it shuts off the gas in its short stroke. Accordingly, the secondary pressure does not rise as in the former case, and a small quantity of gas flows through the orifice 61.

Adjusting of the pressure is done by the pressure adjusting screw 55. When the screw is rotated in, the secondary pressure of the pressure reducing mechanism becomes high and quantity of flow increases. According to this function, a fixed quantity of flow is supplied controlled by the orifice 61 from the spout 6.

As it is difficult to judge whether the gas is flowing or stops if the dial 54 is freely rotatable, it is convenient to do as hereinafter described. One of the dial 54 and the body 1 is provided with a groove and the other with a pin which is fitted in the groove to decide a rotatable angle of the dial 54. The gas flow can be regulated from zero to a fixed quantity by the operation of the dial 54. In the embodiment shown in the drawings, the body 1 is provided with a pin 12 at the top thereof and the dial 54 is provided with an annular groove 56. The annular groove has a common center to one of a rotation of the top plate 57 of the dial 54 and into which the pin 12 is fitted. The pin 12 is inserted in a hole 14 opened at the top 13 of the body 1, after engaging the dial 54 on the body 1 and inserting the pin 12 through a hole 58 which passes through outward and is provided at a portion of the annular groove 56. The relation between the dial 54 and the valve pin 4 is as hereinafter described.

In the state an end portion of the groove 56 contacts with the pin 12 when the dial 54 is rotated in, the passing portion 49 of the valve pin 4 is against the O-ring 49. Afterward the piston 51 and the valve pin 4 repeat opening and shutting off between the primary and the secondary sides to keep a fixed secondary pressure. In case the other end portion of the groove 56 comes in contact with the pin 12 when the dial 54 is rotated back, the shutting off portion 43 of the valve pin 4 closely fits to the O-ring 49 and the gas stops. Accordingly, in this case, quantity of flow of the gas can be controlled continuously from zero to the maximum to be needed, only by rotating the dial 54 in one or opposite direction till the stopping portion, and this is very convenient.

In the drawings, 71 is a V-packing, 72 is a push rod for the valve pin 4 and 73 is an O-ring.

In the portable oxygen inhaler shown in the drawings, to the spout 6 a mask or the like is connected. After the dial 54 is rotated back to shut off the reduced gas passage 33, the cartridge B is pierced by the piercing device. The gas flows in the passage 44 of the valve housing 45 of the valve pin 4 through the gas passage 25 of the needle block 23 of the primary pressure side. But the gas does not flows into the secondary space 11 as the shutting off portion 43 closely fits in the O-ring 49. Hereat, when the dial 54 is rotated downward, the piston 51 is pressed down and the valve pin 4 is also pressed down against the spring 46. When the shutting off portion 43 of the valve pin 4 parts from the O-ring 49 and faces the passage 42, the gas flows into the secondary space 11 and is emitted reduced its pressure by the pressure adjusting spring 52 and fixed its quantity of flow through the spout 6.

The gas emitted may be inhaled. During flowing of the gas, if the pressure in the secondary space 11 rises by some causes, the piston 51 moves upward compressing the spring 52. The valve pin 4 is also pressed up, and the shutting off portion 43 of which closely fits to the O-ring 49. So the gas stops and the pressure in the space 11 is reduced. Therefore the piston 51 falls again and presses the valve pin 4 downward, then the gas begin flowing. If quantity of flow is desired to be controlled, the dial 54 is used. When the dial 51 is rotated downward, the position of the piston 51 moves downward. Then the secondary pressure of the pressure adjusting mechanism rises and quantity of flow increases. The dial 54 being backed, the secondary pressure of the pressure adjusting mechanism falls and quantity of flow is decreased.

In the case of stopping gas supply, the dial 54 is rotated till the other end of the groove 56 comes into contact with the pin 12.

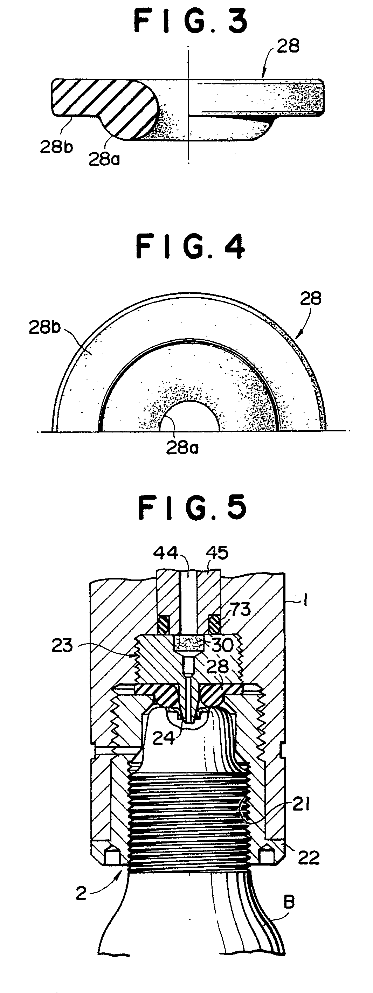

Figures 3 to 5 show a modification of the packing 28 shown in Figure 1. The packing 28 comprises an O-ring portion 28a and a flange portion 28b. The flange portion 28b is less in thickness than the diameter of the O-ring portion in section and is made in one body with the O-ring portion 28a. The flange portion 28b also projects outwardly at right angles with the axial line of the O-ring portion 28b. The packing 28 is made of elastic material such as rubber, plastics, etc.

In this case, as shown in Figure 5, the O-ring 31 may be omitted. In Figure 5, the body 1 is made thick at its medium portion and the screw cylinder 22 is formed like as a nut. The packing 28 is fixed at its flange portion 28b between the lower face of the needle block 23 and the top of the screw cylinder 22.

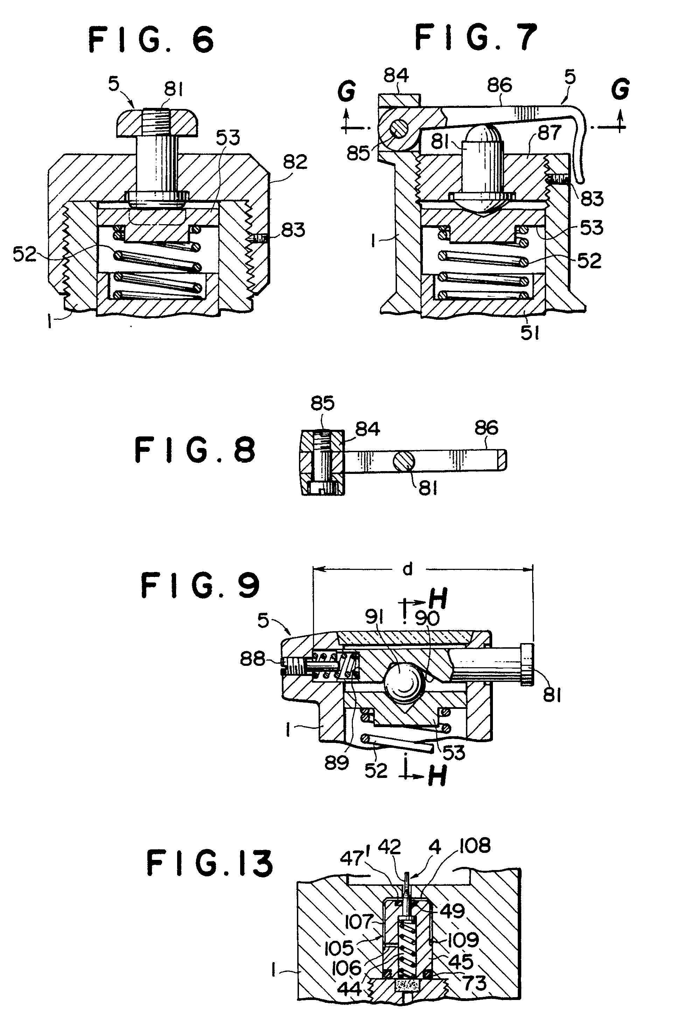

Figures 6 to 12 show embodiments of repective mechanisms to operate the valve pin 4. In these embodiments, as emission of gas is done by means of a push button or a lever, gripping of the body and emitting operation can be done by one hand.

In Figure 6, a push button 81 is used for operating the valve pin 4 ( not shown ) instead of the dial 54 in Figure 1. Also the function by the pressure adjusting screw 55 is replaced by a dial 82 and a set screw 83. The pressure adjusting spring 52 of such a type is employed, as a compressive force becomes maximum when the push button 81 moves lowermost and one becomes zero when uppermost. The push button 81 and the spring seat 53 may be made in one body. When the push button 81 is fully pushed in and fully compresses the pressure adjusting spring 52, the valve pin 4 reaches at its lowermost position. Accordingly, high pressure is necessary to move the valve pin 4 to shut off the gas, so that a large quantity of the gas flows out. On the contrary, when the push button is pushed in a short stroke, the valve pin shuts off the gas even its short upward movement, by a comparatively low pressure, so that a small quantity of the gas flows out.

Figures 7 and 8 show a type of a lever. At the top of the body 1 a bearing 84 is provided, and a lever 86 is pivoted at its one end by a pin 85. The other end of the lever 86 extends towards the opposite side of the top of the body 1 across the center thereof. A screw plug 87 is screwed in the upper end of the body 1, and the push button 81 is is loosely fitted in the plug 87. The upper end of the push button 81 is in contact with the lower plane of the lever 86. The fixing of the screw plug 87 is done by the set screw 83.

In this case, the push button 81 is pushed in by means of the lever 86 instead of pushing directly the push button 81 as shown in Figure 6. As the principle of a lever is applied and a free end of the lever 86 reaches over the side of the body 1, the operation of the inhaler becomes easy.

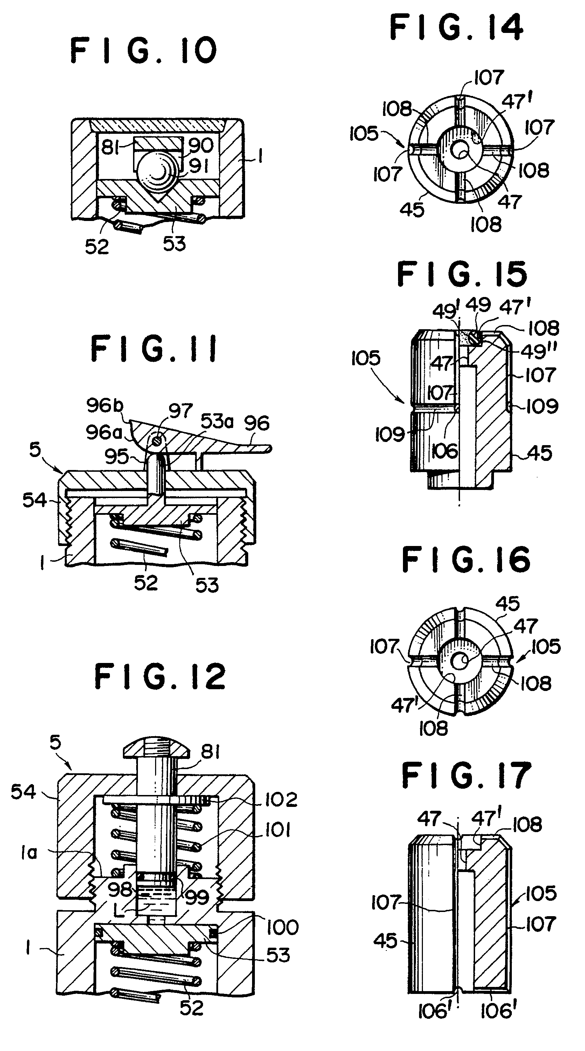

Figures 9 and 10 show a modification of a type of a push button, in which the push rod protrudes outwardly from the side of the body 1. 88 is an adjust pin which is screwed in the side of the body 1 and which can adjust its axial position. By adjusting the axial direction a movable area d of the push rod 81 can be settled.

The push rod 81 is pushed left in Figure 9 till its end 89 comes into contact with the inner end of the adjust pin 88. By this movement a ball 91 fitting in a notch 90 is pressed downwardly by an inclined plane thereof, and the spring seat 53 moves downward till a certain portion.

Figure 11 shows a cam lever type embodiment. In this embodiment, even the cam lever is released after operating the same, the gas supply can be continued. 95 is a bearing formed on the dial 54, and the bearing 95 carries swingably a cam lever 96 by means of a pin 97. At the end of the cam lever 96, cams 96a and 96b are formed. The cam 96a engages the upper end of a protrusion 53a of the spring seat 53, to move the spring seat 53 upward or downward during the swinging motion of the cam lever 96. The cam 96b continues from the end of the cam 96a and locks the spring seat 53 when it engages the upper end of the protrusion 53a. When the right end of the cam lever 96 is moved upward in Figure 11 by one of fingers, the spring seat 53 moves downward, and at last the cam lever 96 stops by the engagement of the upper end of the protrusion 53a with the cam 96b.

Figure 12 is a modification of a push button type. In this embodiment, a cavity 98 is formed in the top 1a of the body 1 and in the cavity 98 liquid L is charged. The push button 81 is fitted in the cavity 98 from the upside, and the cavity 98 is open at its lower end to the spring seat 53. The push button 81 and the spring seat 53 are provided with seal members 99 and 100, respectively. An area to contact with the liquid L of the push button 81 is less than that of the spring seat 53. Thus the force necessary for pushing the push button 81 can be varied.

Between the top 1a and a flange 102 of the push button 81, a spring 101 to urge the push button is installed. If a receiver is provided, the push button 81 can be fitted up at any place, and it is easy to go up the function of operation.

Figures 13 to 17 are respective embodiments in which, at least when the gas is flowing, a primary gas pressure acts at the same time on both sides of the sealing member by providing a leader to the valve housing. One of the sides is a sealing side co-operative with the valve pin and the other is the opposite side. This balances the pressures which act on both sides of the sealing member and resists its deformation, thus the unity of flow of the gas is done.

The valve housing 45 is provided with a leader 105 which passes to the passage 44 at one end and to an annular groove 47' for the seal member 49 at the other.

Figures 13 to 15 show an embodiment of the leader 105. The leader 105 consists of a lateral hole 106, a vertical groove 107 and a lateral groove 108. The lateral hole 106 is provided radially at the medium of the valve housing 45 and the passage 44 is connected to the outside by the lateral hole 106. The vertical groove 107 a also provided in the outer face of the valve housing 45, and it is connected at its lower end to the lateral hole 106 and reaches at the upper end of the valve housing 45. The lateral groove 108 is provided radially in the upper portion of the valve housing 45, and its outer end connected to the vertical groove 107 and its inner end to the annular groove 47'.

If a primary gas pressure exists in the passage 44 and the valve pin 4 is pressed in, the primary gas pressure acts on the side 49' of the seal member 49, which takes charge of opening or closing the gas passage. Also, the primary gas pressure acts on the side 49" opposite to the side 49' of the seal member through the leader 105. As this, the primary gas pressure acts on both sides 49' and 49" of the seal member 49, and the balance between both pressures is obtained. Therefore, even if the pressure which acts on the side 49' of the seal member 49 varies, deformation of the side 49' toward the side 49" can be restrained. Accordingly, a clearance between the passing portion 42 of the valve pin 4 and the side 49' of the seal member 49 is kept constant, so the gas flows in a fixed quantity.

An annular groove 109 is provided connected to the outer end of the lateral hole 106 in the periphery of the valve housing 45, and the vertical grooves 107 which are connected to the annular groove 109 are equally spaced. Further, the upper ends of the vertical grooves 107 are connected to the annular groove 47' through the lateral grooves 108, respectively. In such an assembly, the seal member 49 is to be pressed equally, and it becomes more certain to check changing of the clearance as a passage.

In this case, as the leader 105 is provided to the valve housing 45, the valve housing can be manufactured easily.

Figures 16 and 17 are the cases where the lateral hole 106 is omitted. When the O-ring 73 between the needle block 23 and the valve housing 45 is not needed as a modification of the leader 105, the vertical groove 107 in the periphery is provided in full length of the valve housing 45. At the upper and lower ends of the vertical groove 107, the radial groove 108 and also a radial groove 106' are provided connected to the vertical groove 107, respectively. By this, it is not necessary to make the lateral hole 106, and the valve housing can be manufactured more easily.

According to the present invention, a cartridge piercing device, a valve pin and an adjusting mechanism for a secondary pressure being aligned on the axial line of a cylindrical body, the inhaler becomes small and easy as a whole for carrying and using. Further, the adjusting mechanism being used as an adjuster for pressure and quantity of flow, the assembly becomes simple and the operation becomes easy. Thus according to the present invention there are many remarkable effects.

## Claims

1. A portable oxygen inhaler comprising on the axial line of a cylindrical body (1)
a cartridge access device (2),
a valve housing (45) having a passage (44) for the gas connecting a primary and a secondary space on each side of a valve pin (4), which is movably inserted in the passage (44) and which has a shutting off portion (43) which tends under the pressures of a spring (46) and gas to shut off said passage (44) with a co-operative side (49') of a seal member (49), an adjusting mechanism (5) for the secondary pressure space (11) being in contact at one end of the adjusting mechanism with an end of said valve pin (4) in said secondary space (11), said secondary space being connected to a spout (6) through an orifice (61), the quantity of flow of the gas through said orifice (61) being adjustable by altering the secondary pressure with said adjusting mechanism (5) characterised in that
said cartridge access device (2) has a needle (24) for piercing the cartridge and packing (28) which surrounds the needle (24), said valve housing (45) is mounted in said cartridge access device (2) and a conduit (105) connects the primary gas pressure space with the side (49') of the seal member (49) opposite to said co-operative side (49') whereby the opposite side (49") is subjected to the primary gas pressure so as to equalise the gas pressure on the seal member (49) when the valve is open.

2. A portable oxygen inhaler as claimed in claim 1 wherein said conduit (105) is pointed in the valve housing (45).

3. A portable oxygen inhaler as claimed in claim 1 or claim 2, wherein said packing (28) comprises an O-ring portion (28a) and a flange portion (28b) which is less in thickness than the diameter of the O-ring portion in section, which is made in one body with the O-ring portion (28a), and which projects outwardly at right angles with the axial line of the O-ring portion (28a).

4. A portable oxygen inhaler as claimed in any preceding claim, wherein said adjusting mechanism (5) comprises a piston (51) which is fitted slidably in said secondary pressure space (11) and has a lower surface which contacts with the top of the valve pin (4) at its lower plane, a pressure adjusting spring (52) which is installed between the piston (51) and an upper spring seat (53), and an actuator (5) for the valve pin (4) which is mounted on the upper portion of the cylindrical body (1) and acts on the piston (51) via the spring seat (53).

5. A portable oxygen inhaler as claimed in claim 4, wherein said actuator (5) takes the form of a dial (54) which is screwed in a rotatable fashion on the upper portion of the cylindrical body (1) and a pressure adjusting screw (55) which is screwed in the dial (54) to contact with the spring seat (53) and adjust the secondary pressure, wherein one of the dial (54) and the body (1) is provided with a groove (56) and the other with a pin (12) which is slidably fitted in the groove (56) to decide a rotatable angle of the dial (54), and wherein the gas is controlled continuously from zero to the maximum by moving said pin (12) in said groove (56) from one end to the other end of the groove (56).

6. A portable oxygen inhaler as claimed in claim 4, wherein said actuator (5) takes the form of a dial (82) which is screwed in a rotatable fashion on the upper portion of the cylindrical body (1) to adjust the secondary pressure and a push button (81) through the dial (82) to press the spring seat (53) in for operating the inhaler in an on-off mode.

7. A portable oxygen inhaler as claimed in claim 4, wherein said actuator (5) takes the form of a lever which is pivotally mounted on a bearing member (84) protruded from the upper end of the body (1) and a push button (81) which is movable through a screw plug (87) fixably screwed on the upper inner end portion of the body (1) and which contacts at its upper end with the lever (86) and at its lower end with the spring seat (53).

8. A portable oxygen inhaler as claimed in claim 4, wherein said actuator (5) takes the form of a push rod (81) which protrudes sidewide across the axial line of the body (1) and which has a cam notch (90) to press the spring seat (53) by means of a ball (91) and an adjust pin (88) which is screwed on the side wall of the body (1) and is oriented on the same axial line as the push rod, and the inner end of which is adjustable in its axial direction so as to adjust the stroke of the push rod (81).

9. A portable oxygen inhaler as claimed in claim 4, wherein said actuator (5) takes the form of a cam lever (96) and a dial (54), the cam lever having a profile constituted by two parts, one of which is for pushing down an upward protrusion (53a) from the spring seat (53) during a swinging motion of the cam lever (96) and the other is for locking the upward protrusion (53a) at its lowest position and being pivotally mounted on a bearing member (95) protruded from the top of the dial (54), and the dial being screwed on the upper end of the body (1).

10. A portable oxygen inhaler as claimed in claim 4, wherein said actuator (5) takes the form of a push button (81) and a dial (54) which is screwed on a top portion (1a) of the body (1), the push button (81) fitting through the dial (54) in a cavity (98) in said top portion, the cavity (98) being kept liquid-tight by seal members (99, 100) of the push button (81) and the spring seat (53) and filled with liquid and having a tendency to move upward by means of a compression spring (101) installed between said top portion (1a) and a flange (102) of the push button (81).

**Revendications**

1. Un inhalateur d'oxygène portatif comprenant dans l'axe d'un corps cylindrique (1)
   . un dispositif d'accès à la cartouche (2)
   . un logement de clapet (45) comprenant un passage (44) pour le gaz reliant un espace primaire à un espace secondaire de chaque côté d'un pointeau (4), mobile dans le passage (44) et comportant une partie de fermeture (43) qui, sous la pression du gaz et d'un ressort (46) tend à fermer ledit passage (44) avec une face co-active (49') d'un joint d'étanchéité (49), un mécanisme de réglage (5) de la pression dans l'espace secondaire (11) étant en contact à l'une des extrémités du mécanisme de réglage avec une extrémité dudit pointeau (4) dans ledit espace secondaire (11), ledit espace secondaire étant relié à un bec (6) par l'intermédiaire d'un orifice (61) le débit de gaz passant par ledit orifice (61) étant réglable en modifiant la pression secondaire au moyen dudit mécanisme de réglage (5) caractérisé en ce que

ledit dispositif d'accès à la cartouche (2) comprend une aiguille de perçage (24) de la cartouche et un garnissage (28) qui entoure l'aiguille (24), ledit logement de clapet (45) étant monté sur ledit dispositif d'accès à la cartouche (2) et un conduit (105) reliant l'espace de pression primaire du gaz à la face (49') du joint d'étanchéité (49) opposé audit côté co-actif (49') par lequel la face opposée (49") est soumise à la pression primaire du gaz de manière à équilibrer la pression du gaz sur le joint d'étanchéité (49) lorsque le clapet est ouvert.

2. Un inhalateur d'oxygène portatif selon la revendication 1, dans lequel ledit conduit (105) est dirigé vers le logement de clapet (45).

3. Un inhalateur d'oxygène portatif selon la revendication 1 ou la revendication 2, dans lequel ledit garnissage (28) comprend un joint torique (28a) et une bride (28b) dont l'épaisseur est inférieure au diamètre de la section du joint torique, qui est faite d'un seul tenant avec le joint torique (28a) et qui se dirige vers l'extérieur à angle droit par rapport à l'axe du joint torique (28a).

4. Un inhalateur d'oxygène portatif selon l'une des revendications précédentes, dans lequel ledit mécanisme de réglage (5) comprend un piston (51) coulissant dans ledit espace de pression secondaire (11) et dont la surface inférieure entre en contact avec le sommet du pointeau (4) sur son plan inférieur, un ressort de réglage de pression (52) qui est monté entre le piston (51) et un siège de ressort supérieur (53), et un actionneur (5) du pointeau (4) qui est monté sur la partie supérieure du corps cylindrique (1) et agit sur le piston (51) par l'intermédiaire du siège de ressort (53).

5. Un inhalateur d'oxygène portatif selon la revendication 4, dans lequel ledit actionneur (5) est conçu comme une tête de commande (54), vissée par rotation sur la partie supérieure du corps cylindrique (1) et une vis de réglage de pression (55) vissée dans la tête de commande (54) entrant en contact avec le siège de ressort (53) et réglant la pression secondaire, dans lequel, soit la tête de commande (54) ou le corps (1) comporte une gorge (56) et l'autre partie, un ergot (12) coulissant dans une gorge (56) pour définir l'angle de rotation de la tête de commande (54), et dans lequel le débit du gaz est contrôlé de façon continue, du point zéro au débit maximal, en déplaçant ledit ergot (12) dans ladite gorge (56) d'une extrémité à l'autre de la gorge (56).

6. Un inhalateur d'oxygène portable selon la revendication 4, dans lequel ledit actionneur (5) est réalisé comme une tête de commande (82) vissée bar rotation sur la partie supérieure du corps cylindrique (1) pour régler la pression secondaire et un bouton poussoir (81) passant à travers la tête de commande (82) pour compresser le siège de ressort (53) et faire fonctionner l'inhalateur suivant un système marche/arrêt.

7. Un inhalateur d'oxygène portable selon la revendication 4, dans lequel ledit actionneur (5) est réalisé comme un levier monté pivotant sur un support (84) en saillie de l'extrémité supérieure du corps (1) et un bouton poussoir (81) mobile par l'intermédiaire d'un bouchon à vis (87) vissé sur l'extrémité supérieure intérieure du corps (1), et qui entre en contact à son extrémité supérieure avec le levier (86), et à son extrémité inférieure avec le siège de ressort (53).

8. Un inhalateur d'oxygène portatif selon la revendication 4, dand lequel ledit actionneur (5) est réalisé comme un poussoir (81) s'étendant latéralement par rapport à l'axe du corps (1) et qui comporte une encoche à came (90) destinée à exercer une pression sur le siège de ressort (53) par l'intermédiaire d'une bille (91) et une tige de réglage (88) vissée sur la paroi latérale du corps (1) et dans le même axe que le poussoir (81), et son extrémité intérieure étant réglable axialement de manière à régler la course du poussoir (81).

9. Un inhalateur d'oxygène portatif selon la revendication 4, dans lequel ledit actionneur (5) est réalisé comme un levier à came (96) et une tête de commande (54), le levier à came étant constitué de deux parties, la première partie étant destinée à pousser une saillie (53a) dirigée vers le haut du siège de ressort (53) occasionné par le basculement du levier à came (96), et la seconde étant destinée à bloquer la saillie supérieure (53a) dans sa position inférieure maximale, et pouvant pivoter sur une portée (95) en saillie par rapport au sommet de la tête de commande (54), la tête de commande étant vissée sur l'extrémité supérieure du corps (1).

10. Un inhalateur d'oxygène portatif selon la revendication 4, dans lequel ledit actionneur (5) est réalisé comme un bouton poussoir (81) et une tête de commande (54) vissée sur une partie supérieure (1a) du corps (1), le bouton poussoir (81) étant ajusté dans la tête de commande (54) dans une cavité (98) de ladite partie supérieure, la cavité (98) étant étanchéifiée par les joints d'étanchéité (99, 100) du bouton poussoir (81) et du siège de ressort (53), et remplie d'un liquide et ayant tendance à se déplacer vers le haut grâce à un ressort de compression (101) monté entre ladite partie supérieure (1a) et la bride (102) du bouton poussoir (81).

**Ansprüche**

1. Tragbares Sauerstoffinhaliergerät, das auf der Achse eines zylindrischen Körpers (1) eine Kartuschenzugriffsvorrichtung (2), und ein Ventilgehäuse (45) mit einem Gasdurchgang (44) aufweist, der einen ersten und einen zweiten Raum auf jeder Seite einer Ventilnadel (4) verbindet, die beweglich in den Durchgang (44) eingesetzt ist und die einen Verschlußteil (43) hat, der unter dem Druck einer Feder (46) und eines Gases dazu tendiert, den Durchgang (44) mit einer mitwirkenden Seite (49') eines Dichtgliedes (49) abzuschließen, wobei ein Verstellmechanismus (5) für den zweiten Druckraum (11), an seinem einen Ende ein Ende der Ventilnadel (4) im zweiten Raum (11) berührt, dieser zweite Raum über eine Öffnung (61) mit einer Tülle (6) verbunden ist und die Strömungsmenge des Gases durch die Öffnung (61) durch Verändern des zweiten Druckes mit dem Verstellmechanismus (5) verstellbar ist, dadurch gekennzeichnet, daß die Kartuschenzugriffsvorrichtung (2), eine Nadel (24) zum Durchstechen der Kartusche und eine Dichtung (28) aufweist, welche die Nadel (24) umgibt, das Ventilgehäuse (45) in dieser Kartuschenzugriffsvorrichtung (2) angeordnet ist und ein Kanal (105) den ersten Gasdruckraum mit der Seite (49") des Dichtgliedes (49) gegenüber der mitwirkenden Seite (49') verbindet, wobei die Gegenseite (49") so dem ersten Gasdruck unterworfen ist, daß der Gasdruck auf das Dichtglied (49) ausgeglichen wird, wenn das Ventil offen ist.

2. Tragbares Sauerstoffinhaliergerät nach Anspruch 1, wobei der Kanal (105) in das Ventilgehäuse (45) gekerbt ist.

3. Tragbares Sauerstoffinhaliergerät nach Anspruch 1 oder 2, bei dem die Dichtung (28) einen O-Ringteil (28a) und einen Flanschteil (28b) aufweist, der eine geringere Dicke hat als der Durchmesser des O-Ringteiles im Querschnitt, der in einem Teil mit dem O-Ringteil (28a) gemacht ist und der nach außen im rechten Winkel zur Achse des O-Ringteiles (28a) übersteht.

4. Tragbares Sauerstoffinhaliergerät nach einem der vorhergehenden Ansprüche, bei dem der Verstellmechanismus (5) einen Kolben (51), der verschiebbar in den zweiten Druckraum (11) eingepaßt ist und eine untere Oberfläche hat, die mit ihrer unteren Ebene die Spitze der Ventilnadel (4) berührt, eine Druckeinstellfeder (52), die zwischen dem Kolben (51) und einem oberen Federsitz (53) angebracht ist, und einen Auslöser (5) für die Ventilnadel (4), der am oberen Teil des zylindrischen Körpers (1) angebracht ist und auf den Kolben (51) über den Federsitz (53) wirkt, aufweist.

5. Tragbares Sauerstoffinhaliergerät nach Anspruch 4, bei dem der Auslöser (5) von einer Einstellscheibe (54), die auf drehbare Art auf den oberen Teil des zylindrischen Körpers (1) geschraubt ist, und einer Druckeinstellschraube (55) gebildet wird, die in die Einstellscheibe (54) geschraubt ist, um den Federsitz (53) zu berühren und den zweiten Druck einzustellen, wobei eines der aus Einstellscheibe (54) und Körper (1) bestehenden Teile mit einer Rille (56) und das jeweils andere Teil mit einem Stift (12) versehen ist, der verschiebbar in die Rille (56) eingepaßt ist, um einen Drehwinkel der Einstellscheibe (54) festzusetzen, und wobei das Gas stufenlos von null bis zum Maximum reguliert werden kann, indem der Stift (12) in der Rille (56) von einem Ende zum anderen Ende der Rille (56) bewegt wird.

6. Tragbares Sauerstoffinhaliergerät nach Anspruch 4, bei dem der Auslöser (5) von einer Einstellscheibe (82), die auf drehbare Art auf den oberen Teil des zylindrischen Körpers (1) geschraubt ist, um den zweiten Druck einzustellen, und einem Druckknopf (81), der durch die Einstellscheibe (82) geht, um den Federsitz (53) hineinzudrücken, um das Inhaliergerät an- und auszuschalten, gebildet wird.

7. Tragbares Sauerstoffinhaliergerät nach Anspruch 4, bei dem der Auslöser (5) von einem Hebel, der drehbar auf einem Lagerteil (84) über das obere Ende des Körpers (1) hervorstehend angebracht ist, und einem Druckknopf (81), der beweglich in einem Schraubstöpsel (87) ist, welcher fest in den oberen inneren Endteil des Körpers (1) geschraubt ist, und der mit seinem oberen Ende den Hebel (86) und mit seinem unteren Ende den Federsitz (53) berührt, gebildet wird.

8. Tragbares Sauerstoffinhaliergerät nach Anspruch 4, bei dem der Auslöser (5) von einer Stößelstange (81), die seitwärts über die Achse des Körpers (1) hervorsteht und die eine Mitnehmerkerbe (90) hat, um den Federsitz (53) mittels eines Balles zu drücken, und einem Einstellstift (88) gebildet ist, der in die Seitenwand des Körpers (1) geschraubt und in derselben Achse wie die Stößelstange ausgerichtet ist, und dessen inneres Ende in seiner axialen Richtung verstellbar ist, um den Hub der Stößelstange (81) einzustellen.

9. Tragbares Sauerstoffinhaliergerät nach Anspruch 4, bei dem der Auslöser (5) von einem Mitnehmerhebel (96) und einer Einstellscheibe (54) gebildet wird, wobei der Mitnehmerhebel ein Profil hat, das aus zwei Abschnitten besteht, von denen einer während einer Schwingbewegung des Mitnehmerhebels (96) einen nach oben hervorstehenden Teil (53a) des Federsitzes (53) herunterdrückt und der andere den nach oben hervorstehenden Teil (53a) in seiner niedrigsten Lage sperrt, und der Mitnehmerhebel (96) drehbar auf einem Lagerteil (95) aufgebracht ist, das über das obere Ende der Einstellscheibe (54) vorsteht, und die Einstellscheibe auf das obere Ende des Körpers (1) geschraubt ist.

10. Tragbares Sauerstoffinhaliergerät nach Anspruch 4, bei dem der Auslöser (5) von einem Druckknopf (81) und einer Einstellscheibe (54) gebildet wird, welche auf einen oberen Teil (1a) des Körpers (1) geschraubt ist, wobei der Druckknopf (81) durch die Einstellscheibe (54) in eine Aushöhlung (98) in diesem oberen Teil paßt, die Aushöhlung (98) von Dichtteilen (99, 100) am Druckknopf (81) und am Federsitz (53) flüssigkeitsdicht gehalten wird und mit Flüssigkeit gefüllt ist und der Druckknopf (81) durch die Einrichtung einer Druckfeder (101), die zwischen dem oberen Teil (1a) und einem Flansch (102) des Druckknopfes (81) angebracht ist, eine Bestrebung hat, sich nach oben zu bewegen.

FIG.1

FIG.2

# FIG. 3

28

28b    28a

# FIG. 4

28b    28

28a

# FIG. 5

44    45

73

23    30

28

24

1

21

22

2    B

# FIG. 6

# FIG. 7

# FIG. 8

# FIG. 9

# FIG. 13

# FIG. 10

# FIG. 14

# FIG. 11

# FIG. 15

# FIG. 12

# FIG. 16

# FIG. 17